# FASCICULE DE BREVET EUROPEEN

(11) **EP 1 495 752 B1**
(45) Date de publication et mention de la délivrance du brevet: **16.03.2011**
(21) Numéro de dépôt: 04291746.8
(22) Date de dépôt: 08.07.2004
(51) Int. Cl.: A61K 8/34, A61K 8/39, A61Q 5/10

(54) **Composition tinctoriale liquide exempte d'acide gras et comprenant du 2-méthyl 1,3-propanediol, procédé de teinture et dispositif**
Flüssiges Haarfärbemittel ohne Fettsäure enthaltend 2-Methyl-1,3-propandiol, Haarfärbeverfahren und Kit
Liquid hair dyeing composition without fatty acide comprising 2-methyl-1,3-propanediol, process and kit

(30) Priorité: 11.07.2003 FR 0308545
(43) Date de publication de la demande: 12.01.2005
(73) Titulaire: L'Oréal, 75008 Paris (FR)
(72) Inventeur: Desenne, Patricia, 92270 Bois Colombes (FR)
(74) Mandataire: Wattremez, Catherine

(56) Documents cités:
- EP-A- 0 962 220
- EP-A- 1 179 336
- EP-A- 1 279 395

## Description

La présente invention a pour objet une composition tinctoriale liquide exempte d'acide gras ou de sel d'acide gras, comprenant au moins une base d'oxydation et du 2-méthyl 1,3-propanediol, une composition prête à l'emploi la comprenant, un procédé de teinture de fibres kératiniques humaines la mettant en oeuvre ainsi qu'un dispositif la comprenant.

Il est connu de teindre les fibres kératiniques humaines, et en particulier les cheveux, avec des compositions tinctoriales contenant des précurseurs de colorants d'oxydation, généralement appelés "bases d'oxydation", en particulier des ortho- ou para-phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les bases d'oxydation et d'autre part par les coupleurs, permet l'obtention d'une palette très riche en coloris.

Ces bases d'oxydation et ces coupleurs sont formulés dans des supports permettant leur application sur les fibres kératiniques après mélange avec un agent oxydant.

Il y a essentiellement deux types de supports, des crèmes ou des liquides, différenciés notamment par leur domaine respectif de viscosité et leur composition. En effet, une crème, lorsqu'elle est mélangée à une composition oxydante, doit pouvoir se fluidifier suffisamment pour permettre un mélange homogène des éléments constitutifs de ces deux compositions et une application facile sur les cheveux, sans toutefois perdre trop de viscosité, pour éviter dans la mesure du possible que la composition ne coule hors de la zone à colorer. Par contre, un support sous forme liquide doit pouvoir, une fois mélangé à la composition oxydante, aboutir à une composition dont la viscosité est plus élevée pour permettre une application dans des conditions appropriées.

Comme mentionné auparavant, la présente invention est plus particulièrement dirigée vers les compositions tinctoriales sous forme liquide.
Les supports liquides présentent en effet l'avantage d'être plus simples à conditionner, plus faciles d'utilisation, notamment l'opération de mélange avec une composition oxydante est facilitée.
De plus, de tels supports constituent une alternative intéressante à des supports de type crème qui peuvent poser, dans certains cas, des problèmes de stabilisation de la viscosité lors du stockage.

Bien souvent, les supports liquides comprennent un solvant choisi par exemple parmi les alcools et leurs éthers.
Si de tels supports permettent d'obtenir des compositions tinctoriales, qui une fois mélangées à des compositions oxydantes, donnent de bons résultats, il est toutefois souhaité d'en améliorer encore les performances.
Il a par ailleurs été constaté que la présence de savons d'acides gras pouvait avoir comme conséquence d'entraîner des désagréments cosmétiques ou encore de limiter la gamme de pH d'utilisation de la composition.
Enfin, dans certains cas, les compositions tinctoriales peuvent avoir, à basse température, un aspect trouble, dû à une homogénéité insuffisante de la composition. La conséquence de ce fait est qu'à plus ou moins long terme, selon les conditions de stockage, un déphasage de la composition tinctoriale est observé.

La présente invention a donc pour objet de proposer des compositions tinctoriales sous forme liquide pour lesquelles on n'observe pas tous ces inconvénients et qui permettent d'aboutir à des résultats de coloration toujours satisfaisants, voire améliorés.

Ces buts et d'autres sont atteints par la présente invention qui a pour premier objet une composition tinctoriale sous forme liquide, exempte d'acide gras ou de sels d'acide gras, comprenant au moins un colorant d'oxydation et du 2-méthyl 1,3-propanediol.

Un autre objet de l'invention consiste en un procédé de teinture de fibres kératiniques humaines, consistant à appliquer sur lesdites fibres la composition tinctoriale précitée et une composition oxydante comprenant au moins un agent oxydant, la composition oxydante étant mélangée au moment de l'emploi à la composition tinctoriale ou appliquée séquentiellement sans rinçage intermédiaire.

Elle a enfin pour objet un dispositif à plusieurs compartiments pour la teinture de fibres kératiniques humaines, comportant au moins un premier compartiment contenant la composition tinctoriale, et un deuxième compartiment contenant une composition oxydante comprenant au moins un agent oxydant.

Ainsi, la composition tinctoriale selon l'invention est stable au stockage en ce sens que sa viscosité ne change pas de manière significative et que l'on observe pas de déphasage dans le temps.

En outre, la composition obtenue après mélange avec une composition oxydante, présente une viscosité adaptée lors de l'application, viscosité qui est conservée pendant la durée de pose du produit sur les fibres.
Enfin, les colorations obtenues avec la mise en oeuvre de telles compositions sont intenses, chromatiques, peu sélectives, et les propriétés cosmétiques des fibres résultantes ne sont pas dégradées de manière substantielle.

Mais d'autres buts et caractéristiques de la présente invention apparaîtront plus clairement à la lecture de la description et des exemples qui vont suivre.

Dans la description, à moins d'une indication différente, les bornes de gammes de valeurs sont considérées comme comprise dans ces gammes.

Ainsi que cela a été indiqué auparavant, la composition tinctoriale selon l'invention se présente sous forme liquide. Il est à noter que la composition tinctoriale ne comprend pas d'agent oxydant.
Plus particulièrement, au sens de l'invention, par composition sous forme liquide on désigne une composition qui, à une température de 25°C, sous 1 atmosphère, présente une viscosité d'au plus 150 cPs, mesurée avec un rhéomètre RhéoStress 1, sous un taux de cisaillement de 200 s⁻¹.

Par ailleurs, selon une caractéristique avantageuse de l'invention, la composition tinctoriale est un liquide limpide. Plus particulièrement, la composition se présente sous la forme d'un liquide isotrope transparent. La composition présente plus précisément, une turbidité comprise entre 60 et 600 NTU et de préférence entre 70 et 400 NTU (mesure effectuée avec un turbidimètre portatif HACH - Modèle 2100 P à 25°C).

La composition selon l'invention est par ailleurs exempte d'acide gras ou de sel d'acide gras.
Par acide gras, on entend les acides carboxyliques comprenant un radical hydrocarboné, saturé ou non, linéaire ou ramifié, comprenant 8 à 30 atomes de carbone, éventuellement porteur d'un ou plusieurs radicaux hydroxyles.
Par sel d'acide gras, on entend plus particulièrement les sels de métaux alcalins ou alcalino-terreux, des acides précités, comme les sels de sodium, de potassium, de magnésium.

Conformément à une caractéristique importante, la composition tinctoriale selon l'invention comprend du 2-méthyl 1,3-propanediol.

La teneur en 2-méthyl 1,3-propanediol représente de préférence de 0,5 à 20 % en poids par rapport au poids de la composition tinctoriale. Selon un mode de réalisation particulier de l'invention, la teneur en 2-méthyl 1,3-propanediol représente de 5 à 20 % en poids par rapport au poids de la composition tinctoriale, et de manière encore plus préférentielle de 10 à 18 % en poids de la composition tinctoriale.

La composition peut en outre comprendre au moins un polyol présentant au moins deux radicaux hydroxyle, différent du 2-méthyl 1,3-propanediol et/ou un monoéther ou polyéther d'un polyol ; le polyol ou la partie polyol du mono- ou polyéther comprenant un radical hydrocarboné, saturé ou non, linéaire ou ramifié comprenant 3 à 50 atomes de carbone, avantageusement de 3 à 10 atomes de carbone, éventuellement interrompu par un ou plusieurs atomes d'oxygène.

Avantageusement, ledit polyol est choisi parmi le pinacol (2,3-diméthyl 2,3-butanediol), le glycérol (1,2,3 propanetriol), le 1,2,3-butanetriol, le 3-méthyl-1,3,5-pentanetriol, le 1,2,4-butanetriol, le propylèneglycol, le dipropylèneglycol, le 1,5-pentanediol, le 3-méthyl-1,5-pentanediol, le néopentylglycol (2,2-diméthyl-1,3-propanediol), l'isoprène glycol (3-méthyl-1,3-butanediol) et l'hexylèneglycol (2-méthyl 2,4-pentanediol), ou leurs mélanges.

Selon un mode de réalisation préféré de l'invention, le polyol, s'il est présent, est choisi parmi le glycérol, le propylèneglycol, le dipropylèneglycol, l'hexylèneglycol, ou leurs mélanges.

Les mono- ou poly-éthers de polyols peuvent comprendre ou non un ou plusieurs groupements hydroxyle. Le ou les radicaux, identiques ou différents, bloquant la ou les fonctions hydroxyle du polyol comprennent, chacun, plus particulièrement de 1 à 30 atomes de carbone. Conformément à un mode de réalisation de l'invention, le ou les radicaux bloquant la ou les fonctions hydroxyle correspondent à des radicaux alkyle. De préférence le nombre total d'atomes de carbone de chacun de ces éthers est compris entre 4 et 50.

A titre illustratif, la teneur en polyol et/ou de mono- et/ou poly- éther dudit polyol, au cas où ce type de composé est présent, représente de 0,1 à 20 % en poids par rapport au poids de la composition tinctoriale, et de préférence de 0,2 à 10 % en poids par rapport au poids de la composition tinctoriale.

Enfin, toujours au cas où il est présent, la proportion de polyol et/ou de mono- et/ou poly-éther dudit polyol dans la composition tinctoriale est plus particulièrement telle que le rapport pondéral 2-méthyl 1,3-propanediol / polyol autre que le 2-méthyl 1,3-propanediol et/ou mono- ou poly-éther de polyol est supérieur à 1, de préférence inférieur ou égal à 10.

La composition peut aussi comprendre au moins un monoalcool à chaîne courte. Plus précisément, ledit monoalcool comprend une chaîne alkyle, linéaire ou ramifiée, comprenant de 2 à 8 atomes de carbone. A titre d'exemples de composés de ce type on peut citer notamment l'éthanol, l'isopropanol, seuls ou en mélanges.

Lorsqu'un monoalcool à chaîne courte est présent dans la composition tinctoriale, sa teneur est comprise entre 0,1 et 20 % en poids par rapport au poids de la composition tinctoriale, de préférence entre 0,2 et 20 % en poids par rapport au poids de la composition tinctoriale.

Il est à noter que les polyols, éthers de polyols et monoalcools additionnels sont présents en quantité telle dans la composition tinctoriale, que cette dernière est un liquide limpide au sens indiqué auparavant.

Il est à noter que la composition tinctoriale selon l'invention comprend, de manière particulièrement avantageuse, de l'eau.

La composition comprend en outre au moins un colorant d'oxydation.

Plus précisément, le colorant d'oxydation est choisi parmi les bases d'oxydation et/ou les coupleurs.

Les bases d'oxydation classiquement mises en oeuvre dans le domaine de la coloration des fibres kératiniques peuvent être présentes dans la composition selon l'invention.
Ainsi, les bases d'oxydation sont choisies parmi les orthophénylènediamines, les paraphénylènediamines, les bases doubles, les orthoaminophénols, les paraaminophénols, les bases hétérocycliques, ainsi que leurs sels d'addition avec un acide ou un agent alcalin, seuls ou en mélanges.

On peut notamment citer :
**(I)** les paraphénylènediamines de formule (I) suivante et leurs sels d'addition avec un acide : 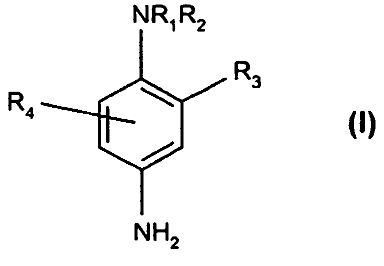 dans laquelle :
   R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido ;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylamino- alcoxy en C₁-C₄,
   R₄ représente un atome d'hydrogène, d'halogène ou un radical alkyle en C₁-C₄.
   Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-para-phénylène diamine, la 4-amino-N,N-diéthyl-3-méthyl-aniline, la N,N-bis-(β-hydroxyéthyl)-paraphénylène diamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, la 2-méthyl-1-N-β-hydroxyéthyl-paraphénylènediamine, la N-(4-aminophényl)-3-hydroxy-pyrrolidine, et leurs sels d'addition avec un acide ou un agent alcalin.
   Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide ou un agent alcalin.
**(II)** Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (II) suivante, et leurs sels d'addition avec un acide ou un agent alcalin : 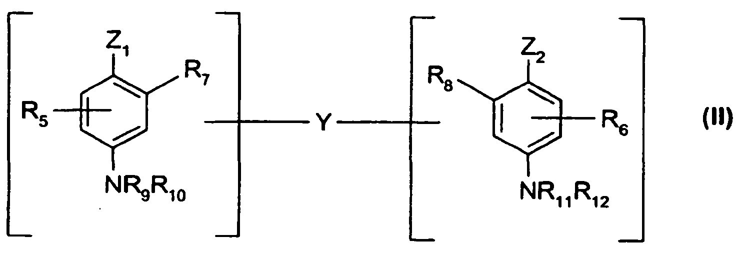 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (II) ne comportent qu'un seul bras de liaison Y par molécule.
   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylène diamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxa octane, et leurs sels d'addition avec un acide ou un agent alcalin.
   Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
**(III)** les paraaminophénols répondant à la formule (III) suivante, et leurs sels d'addition avec un acide ou un agent alcalin : 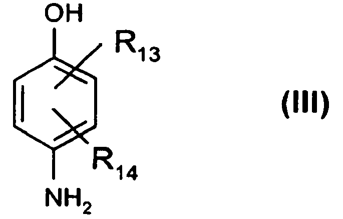 dans laquelle :
   R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).
   Parmi les paraaminophénols de formule (III) ci-dessus, on peut plus particulièrement citer le paraaminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide ou un agent alcalin.
**(IV)** les orthoaminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide ou un agent alcalin.
**(V)** Les bases hétérocycliques utilisables à titre de bases d'oxydation dans les compositions tinctoriales conformes à l'invention sont plus particulièrement choisies parmi les dérivés pyridiniques, les dérivés pyrimidiniques, les dérivés pyrazoliques, et leurs sels d'addition avec un acide ou un agent alcalin.

Parmi les dérivés pyridiniques, on peut plus particulièrement citer les composés décrits par exemple dans les brevets GB 1026978 et GB 1153196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide ou un agent alcalin.

Parmi les dérivés pyrimidiniques, on peut plus particulièrement citer les composés décrits par exemple dans les brevets DE 2359399 ou JP 88169571, JP 9110659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diamino pyrimidine, la 2,5,6-triaminopyrimidine, et les dérivés pyrazolo-pyrimidiniques tels ceux mentionnés dans la demande de brevet FR 2750048 et parmi lesquels on peut citer la pyrazolo-[1,5-a]-pyrimidine-3,7-diamine, la 2,5-diméthyl-pyrazolo-[1,5-a]-pyrimidine-3,7-diamine, la pyrazolo-[1,5-a]-pyrimidine-3,5-diamine, la 2,7-diméthyl-pyrazolo-[1,5-a]-pyrimidine-3,5-diamine, le 3-amino-pyrazolo-[1,5-a]-pyrimidin-7-ol, le 3-amino-pyrazolo-[1,5-a]-pyrimidin-5-ol, le 2-(3-amino pyrazolo-[1,5-a]-pyrimidin-7-ylamino)-éthanol, le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl-pyrazolo-[1,5-a]-pyrimidine-3,7-diamine; la 2,6-diméthyl-pyrazolo-[1,5-a]-pyrimidine-3,7-diamine, la 2,5,N7, N7-tetraméthyl-pyrazolo-[1,5-a]-pyrimidine-3,7-diamine, la 3-amino-5-méthyl-7-imidazolylpropylamino-pyrazolo-[1,5-a]-pyrimidine, et leurs sels d'addition et leurs formes tautomères, lorsqu'il existe un équilibre tautomérique, et leurs sels d'addition avec un acide ou un agent alcalin.

Parmi les dérivés pyrazoliques, on peut plus particulièrement citer les composés décrits dans les brevets DE 3843892, DE 4133957 et demandes de brevet WO 94/08969, WO 94/08970, FR 2733749, FR 2817551 et DE 19543988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-(β-méthoxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide ou un agent alcalin.

Selon la présente invention, les bases d'oxydation représentent habituellement de 0,0005 à 12% en poids par rapport au poids total de la composition tinctoriale, de préférence, de 0,005 à 8% en poids par rapport au poids de la composition tinctoriale.

En ce qui concerne les coupleurs éventuellement présents dans la composition tinctoriale selon l'invention, ils sont choisis plus spécialement parmi les coupleurs généralement mis en oeuvre dans le domaine de la coloration des fibres kératiniques humaines.
A titre d'exemples, on peut citer notamment les métaaminophénols, les métaphénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide ou un agent alcalin.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 2-chloro-3-amino-6-méthyl-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxybenzène, le 1,3-diaminobenzène, le 1,3-bis-(2,4-diaminophénoxy)-propane, le sésamol, le 1-amino-2-méthoxy-4,5-méthylènedioxybenzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide ou un agent alcalin.

Lorsqu'ils sont présents, ces coupleurs représentent avantageusement de 0,0001 à 10% en poids par rapport au poids de la composition, de préférence de 0,005 à 5% en poids par rapport au poids de la composition tinctoriale.

D'une manière générale, les sels d'addition avec un acide des bases d'oxydation et coupleurs sont notamment choisis parmi les chlorhydrates, les bromhydrates, les sulfates, les citrates, les succinates, les tartrates, les tosylates, les benzènesulfonates, les lactates et les acétates.

Les sels d'addition avec un agent alcalin sont notamment choisis parmi les sels d'addition avec les métaux alcalins ou alcalino-terreux, avec l'ammoniaque, avec les amines organiques dont les alcanolamines et les composés de formule suivante : 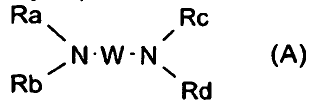 dans laquelle W est un reste propylène éventuellement substitué par un groupement hydroxyle ou un radical alkyle en C₁-C₆ ; Rₐ, R_{b}, R_{c} et R_{d}, identiques ou différents, représentent un atome d'hydrogène, un radical alkyle en C₁-C₆ ou hydroxyalkyle en C₁-C₆.

La composition tinctoriale selon l'invention peut de plus comprendre au moins un colorant direct. Celui-ci peut être choisi parmi les composés ioniques, de préférence cationiques, ou non ioniques.
A titre d'exemples de tels colorants, on peut notamment citer les colorants benzéniques nitrés, les colorants azoïques, les colorants anthraquinoniques, naphtoquinoniques ou benzoquinoniques, les colorants indigoïdes, ou les colorants dérivés du triarylméthane, seuls ou en mélanges.

Lorsqu'ils sont présents, les colorants directs représentent 0,0005 à 12 % en poids du poids de la composition tinctoriale, de préférence de 0,005 à 6 % en poids du poids de la composition tinctoriale.

Selon un mode de réalisation particulier de l'invention, la composition comprend au moins un tensioactif non ionique, cationique, amphotère ou zwittérionique.

Les agents tensioactifs non ioniques sont des composés bien connus en soi (voir notamment à cet égard "Handbook of Surfactants" par M.R. PORTER, éditions Blackie & Son (Glasgow and London), 1991, pp 116-178).
A titre d'exemples, on peut citer sans intention de s'y limiter, les alcools gras polyalcoxylés ou polyglycérolés, les alpha-diols, les alkylphénols en C₈-C₁₈ polyalcoxylés, le nombre de groupements oxyde d'alkylène (oxyde d'éthylène et/ou oxyde de propylène) étant notamment compris entre 2 et 50, le nombre de groupements glycérol étant avantageusement compris enter 1 et 20.
On peut également citer les copolymères d'oxyde d'éthylène et de propylène, les condensats d'oxyde d'éthylène et de propylène sur des alcools gras ; les amides gras polyalcoxylés, ayant plus particulièrement de 2 à 30 moles d'oxyde d'alkylène, de préférence d'oxyde d'éthylène ; les amides gras polyglycérolés, comportant en moyenne 1 à 5 groupements glycérol et en particulier 1,5 à 4 ; les esters d'acides gras du sorbitan oxyéthylénés, ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.
Il est à noter que les composés gras précités comprennent habituellement un radical hydrocarboné en C₇-C₃₀, de préférence en C₇-C₁₈, linéaire ou ramifié, saturé ou non.

Les agents tensioactifs amphotères ou zwitterioniques, peuvent être notamment des dérivés d'amines secondaires ou tertiaires aliphatiques, dans lesquels le radical aliphatique est une chaîne linéaire ou ramifiée comportant 7 à 18 atomes de carbone et contenant au moins un groupe anionique hydrosolubilisant (par exemple carboxylate, sulfonate, sulfate, phosphate ou phosphonate) ; on peut citer encore les alkyl (C₃-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination Miranol, tels que décrits dans les brevets US 2528378 et US 2781354 et classés dans le dictionnaire CTFA, 3ème édition, 1982, sous les dénominations Amphocarboxyglycinates et Amphocarboxypropionates de structures respectives : R-CONHCH₂CH₂N(R')(R")(CH₂COO-) dans laquelle : R désigne un radical alkyle d'un acide R-COOH présent dans l'huile de coprah hydrolysée, un radical heptyle, nonyle ou undécyle, R' désigne un groupement β-hydroxyéthyle et R" un groupement carboxyméthyle ; et R"'-CONHCH₂CH₂-N(B)(C) dans laquelle : B représente -CH₂CH₂OX', C représente -(CH₂)_{z}-Y', avec z = 1 ou 2, X' désigne le groupement -CH₂CH₂-COOH ou un atome d'hydrogène, Y' désigne -COOH ou le radical -CH₂-CHOH-SO₃H, R"' désigne un radical alkyle d'un acide R"'-COOH présent dans l'huile de coprah ou dans l'huile de lin hydrolysée, un radical alkyle plus particulièrement en C₇-C₁₈, notamment en C₇, C₉, C₁₁ ou C₁₃, un radical alkyle en C₁₇ et sa forme iso, un radical C₁₇ insaturé.

Ces composés sont classés dans le dictionnaire CTFA, 1993, sous les dénominations Disodium Cocoamphodiacetate, Disodium Lauroamphodiacetate, Disodium Caprylamphodiacetate, Disodium Capryloamphodiacetate, Disodium Coco-amphodi propionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale Miranol® C2M concentré par la société Rhodia Chimie.

Parmi les tensioactifs cationiques on peut citer en particulier les sels d'amines grasses, notamment en C₇-C₃₀, de préférence en C₇-C₁₈, primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

La teneur en tensioactifs, s'ils sont présents, représente de 0,01 à 40 % en poids par rapport au poids de la composition tinctoriale, et de préférence de 0,1 à 30 % en poids par rapport au poids de la composition tinctoriale

La composition tinctoriale peut également contenir des agents épaississants choisis avantageusement parmi les amides d'acides gras en C₈-C₃₀, éventuellement polyalcoxylés, parmi les polymères, et de préférence parmi les polymères associatifs non ioniques, anioniques, amphotères ou cationiques.

S'ils sont présents, leur teneur varie en général entre 0,05 et 20 % en poids par rapport au poids de la composition tinctoriale, de préférence entre 0,1 et 15 % en poids par rapport à la même référence.

La composition peut aussi comprendre un ou plusieurs agents réducteurs ou antioxydants choisis par exemple parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, ou leurs mélanges.

Lorsqu'ils sont utilisés, leur teneur est habituellement comprise entre 0,05 et 3% en poids par rapport au poids de la composition tinctoriale.

La composition tinctoriale selon l'invention peut en outre comprendre des additifs classiquement mis en oeuvre dans le domaine, comme par exemple des polymères substantifs cationiques ou amphotères, des polymères associatifs anioniques, non ioniques ou cationiques ; des agents séquestrants tels que l'EDTA et l'acide étidronique ; des filtres UV ; des cires ; des silicones volatiles ou non, cycliques ou linéaires ou ramifiées, organomodifiées (notamment par des groupements amines) ou non ; des conservateurs ; des céramides, des pseudocéramides ; des huiles végétales, minérales ou de synthèse ; les vitamines ou provitamines comme le panthénol, des opacifiants, des parfums, etc....

Le pH de la composition tinctoriale selon l'invention est plus particulièrement compris entre 4 et 11.

Il peut être ajusté à la valeur désirée au moyen d'agents acidifiants ou alcalinisants habituellement utilisés dans le domaine.
Ainsi, parmi les agents acidifiants, on peut citer, sans intention de s'y limiter, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.
En ce qui concerne les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (A) détaillée auparavant.

Un autre objet de la présente invention est constitué par une composition prête à l'emploi comprenant la composition tinctoriale qui vient d'être décrite et au moins un agent oxydant.

Plus particulièrement, l'agent oxydant est choisi parmi le peroxyde d'hydrogène, le peroxyde d'urée, les bromates ou ferricyanures de métaux alcalins, les persels tels que les perborates et les persulfates, les enzymes telles que les peroxydases et les oxydo-réductases à deux ou à quatre électrons, seuls ou en mélanges.
Le peroxyde d'hydrogène est de préférence mis en oeuvre.
Cet agent oxydant est avantageusement constitué par une solution de peroxyde d'hydrogène dont le titre peut varier, plus particulièrement, d'environ 1 à 40 volumes, et encore plus préférentiellement d'environ 5 à 40 volumes.

Le pH de la composition prête à l'emploi varie plus particulièrement entre 4 et 11 et est ajusté, si nécessaire, au moyen d'un agent acidifiant ou alcalinisant.

De plus, la composition prête à l'emploi se présente plus particulièrement sous la forme d'une composition de viscosité plus élevée que celle de la composition tinctoriale.
Enfin, la composition prête à l'emploi a de manière avantageuse l'aspect d'un gel.

La présente invention a de même pour objet un procédé de teinture de fibres kératiniques humaines, consistant à appliquer sur lesdites fibres la composition tinctoriale selon l'invention, et une composition oxydante comprenant au moins un agent oxydant ; la composition oxydante étant mélangée au moment de l'emploi à la composition tinctoriale ou appliquée séquentiellement sans rinçage intermédiaire.

La composition oxydante comprend, dans un milieu approprié pour la teinture, au moins un agent oxydant.

Selon la présente invention, on entend par fibres kératiniques humaines, les cheveux, les cils, les sourcils, la moustache, notamment.

La température est de manière classique comprise entre la température ambiante (15 à 25°C) et 80°C et plus particulièrement entre 15 et 40°C.

Par ailleurs, la composition tinctoriale et la composition oxydante sont appliquées pendant le temps nécessaire au développement de la coloration. Celui-ci est en général d'environ 3 à 60 minutes et plus particulièrement d'environ 5 à 40 minutes.

Une fois le temps de pause écoulé, on rince habituellement les fibres, on les lave éventuellement au shampooing, on les rince et on les sèche.

Selon une variante du procédé de l'invention, il comporte une étape préliminaire consistant à stocker sous forme séparée, d'une part, la composition tinctoriale selon l'invention et, d'autre part, une composition oxydante, puis à procéder à leur mélange au moment de l'emploi avant d'appliquer ce mélange sur les cheveux pendant un temps suffisant pour développer la coloration désirée, après quoi on rince, on lave éventuellement au shampooing, on rince à nouveau et on sèche.

Les exemples qui vont suivre sont donnés pour illustrer la présente invention sans toutefois en limiter la portée.

### EXEMPLE 1

On prépare la composition suivante (Les quantités sont données en % en matière active) :

| | |
|---|---|
| alcool laurylique oxyéthyléné (3 OE) | 6 |
| alcool laurylique oxyéthyléné (2 OE) | 4 |
| alcool decylique oxyéthyléné (5 OE) | 9 |
| monoéthanolamide d'acide alkyl (C13/C15 70/30 50 % linéaire) éther carboxylique (2 OE) | 12 |
| lauryl amino succinamate de diéthylaminopropyle, sel de sodium en solution aqueuse | 2,5 |
| alcool éthylique 96 degrés dénaturé | 4 |
| 2-méthyl 1,3-propanediol désodorisé | 13 |
| propylène glycol | 5 |
| 1-méthyl-2-hydroxy-4-amino-benzène | 2,5 |
| 1,3-dihydroxybenzène (résorcinol) | 0,01 |
| 1-hydroxy-4-amino-benzène | 0,86 |
| 1,4-diamino-benzène | 0,95 |
| 3-méthyl-1-phényl-5-pyrazolone | 0,15 |
| 1-méthyl-2-hydroxy-4-béta-hydroxyéthylamino-benzène | 0,47 |
| polycondensat diéthyl diméthyl éthylènediamine / dibromo 1,3-propylène en solution aqueuse a 50 % | 2,5 |
| monoéthanolamine pure | 3,5 |
| réducteur, antioxydant | qs |
| séquestrant | qs |
| Parfum | 0,5 |
| eau désionisée | qs 100 |

La composition est limpide et stable.

La composition est mélangée avec une composition aqueuse d'eau oxygénée à 20 volumes, puis appliquée sur une mèche de cheveux naturels à 90 % de blanc. Le rapport de bain est de 1/10.
Après un temps de pause de 20 minutes, la mèche est shampooinée, rincée puis séchée.
La mèche obtenue est colorée en rouge.

### EXEMPLE 2

On prépare la composition suivante (Les quantités sont données en % en matière active) :

| | |
|---|---|
| alcool oléique polyglycérolé (4 PG) | 3,9 |
| alcool décylique oxyéthyléné (3 OE) | 8,1 |
| alcool décylique oxyéthyléné (5 OE) | 7,2 |
| monoéthanolamide d'acide alkyl (C13/C15 70/30 50 % linéaire) éther carboxylique (2 OE) | 10 |
| lauryl amino succinamate de diéthylaminopropyle, sel de sodium en solution aqueuse | 2,5 |
| 2-méthyl 1,3-propanediol désodorisé | 12 |
| propylène glycol | 10 |
| 1-méthyl-2-hydroxy-4-amino-benzène | 1,2 |
| 1,3-dihydroxybenzène (résorcinol) | 0,094 |
| 1-hydroxy-4-amino-benzène | 0,24 |
| 1,4-diamino-benzène | 0,3 |
| 3-méthyl-1-phényl-5-pyrazolone | 0,15 |
| 1-méthyl-2-hydroxy-4-béta-hydroxyéthylamino-benzène | 0,18 |
| 6-hydroxyindole | 0,05 |
| polycondensat diéthyl diméthyl éthylènediamine / dibromo 1,3-propylène en solution aqueuse a 50 % | 1,5 |
| monoéthanolamine pure | 3,4 |
| réducteur, antioxydant | qs |
| séquestrant | qs |
| parfum | 0,5 |
| eau désionisée | qs 100 |

La composition obtenue est limpide et stable.

La composition est mélangée avec une composition aqueuse d'eau oxygénée à 20 volumes, puis appliquée sur une mèche de cheveux naturels à 90% de blanc. Le rapport de bain est de 1/10.

Après un temps de pause de 20 minutes, la mèche est shampooinée, rincée puis séchée. La mèche obtenue est colorée en rouge.

## Revendications

1. Composition tinctoriale sous forme liquide dont la viscosité est au plus 150 mPas (150 cps) à une température de 25°C, sous une atmosphère, mesurée avec un rhéomètre RhéoStress 1, sous un taux de cisaillement de 200 s⁻¹, exemple d'acide gras ou de sels d'acide gras, comprenant au moins un colorant d'oxydation et du 2-méthyl 1,3-propanediol.

2. Composition selon la revendication précédente, **caractérisée en ce que** la teneur en 2-méthyl 1,3-propanediol représente 0,5 à 20 % en poids, plus particulièrement de 5 à 20 % en poids, et de préférence de 10 à 18 % en poids de la composition tinctoriale.

3. Composition selon l'une quelconque des revendications précédentes, **caractérisée en ce qu'**elle comprend au moins un polyol présentant au moins deux radicaux hydroxyle, différent du 2-méthyl 1,3-propanediol et/ou un monoéther ou polyéther d'un polyol, le polyol et le mono- ou polyéther comprenant un radical hydrocarboné, saturé ou non, linéaire ou ramifié comprenant 3 à 50 atomes de carbone, avantageusement de 3 à 10 atomes de carbone, éventuellement interrompu par un ou plusieurs atomes d'oxygène.

4. Composition selon la revendication précédente, **caractérisé en ce que** le polyol est choisi parmi le pinacol, le glycérol, le 1,2,3-butanetriol, le 3-méthyl-1,3,5-pentanetriol, le 1,2,4-butanetriol, le propylèneglycol, le dipropylèneglycol, le 1,5-pentanediol, le 3-méthyl-1,5 pentanediol, le néopentylglycol, l'isoprène glycol et l'hexylèneglycol, ou leurs mélanges.

5. Composition selon la revendication précédente, **caractérisée en ce que** le polyol est choisi parmi le glycérol, le propylèneglycol, le dipropylèneglycol, l'hexylèneglycol, ou leurs mélanges.

6. Composition selon l'une quelconque des revendications 3 à 5, **caractérisée en ce que** la proportion de polyol est telle que le rapport pondéral 2-méthyl 1,3-propanediol / polyol est supérieur à 1, de préférence inférieur ou égal à 10.

7. Composition selon l'une quelconque des revendications précédentes, **caractérisée en ce qu'**elle comprend au moins un monoalcool à chaîne courte, plus particulièrement au moins un monoalcool comprenant une chaîne alkyle, linéaire ou ramifiée, comprenant de 2 à 8 atomes de carbone.

8. Composition selon la revendication précédente, **caractérisée en ce que** la teneur en monoalcool à chaîne courte est comprise entre 0,1 et 20 % en poids par rapport au poids de la composition tinctoriale, de préférence entre 0,2 et 20 % en poids par rapport au poids de la composition tinctoriale.

9. Composition selon l'une quelconque des revendications précédentes, **caractérisée en ce que** le colorant d'oxydation est choisi parmi les bases d'oxydation et/ou les coupleurs.

10. Composition selon la revendication précédente, **caractérisée en ce que** la base d'oxydation est choisie parmi les ortho-phénylènediamines, les para-phénylènediamines, les bases doubles, les ortho-aminophénols, les para-aminophénols, les bases hétérocycliques, ainsi que leurs sels d'addition avec un acide ou un agent alcalin, seuls ou en mélanges.

11. Composition selon la revendication précédente, **caractérisée en ce que** la teneur en bases d'oxydation est comprise entre 0,0005 et 12% en poids par rapport au poids de la composition tinctoriale, de préférence entre 0.005 et 8% en poids par rapport au poids de la composition tinctoriale.

12. Composition selon la revendication 9, **caractérisée en ce que** les coupleurs sont choisis parmi les méta-aminophénols, les méta-phénylènediamines, les métadiphénols, les naphtols, les coupleurs hétérocycliques, leurs sels d'addition avec un acide ou un agent alcalin.

13. Composition selon la revendication précédente, **caractérisée en ce que** la teneur en coupleurs est comprise entre 0,0001 et 10 % en poids par rapport au poids de la composition tinctoriale, de préférence entre 0,005 et 5 % en poids par rapport au poids de la composition tinctoriale.

14. Composition selon l'une quelconque des revendications précédentes, **caractérisée en ce qu'**elle comprend au moins un colorant direct.

15. Composition selon la revendication précédente, **caractérisée en ce que** le colorant direct est choisi parmi les colorants benzéniques nitrés, les colorants azoïques, les colorants anthraquinoniques, naphtoquinoniques ou benzoquinoniques, les colorants indigoïdes, ou les colorants dérivés du triarylméthane, seuls ou en mélanges.

16. Composition selon l'une des revendications 14 ou 15, **caractérisée en ce que** la teneur en colorant direct représente de 0,0005 à 12 % en poids, par rapport au poids de la composition tinctoriale, de préférence de 0,005 à 6 % en poids, par rapport au poids de la composition tinctoriale.

17. Composition selon rune quelconque des revendications précédentes, **caractérisée en ce qu'**elle comprend au moins un tensioactif non ionique, cationique, amphotère ou zwittérionique.

18. Composition selon la revendication précédente, **caractérisée en ce que** la teneur en tensioactif est comprise entre 0.01 et 40% en poids, par rapport au poids de la composition tinctoriale, de préférence entre 0,1 et 30% du poids par rapport au poids de la composition tinctoriale.

19. Composition selon l'une quelconque des revendications précédentes, **caractérisée en ce que** la composition comprend de l'eau.

20. Composition selon l'une quelconque des revendications précédentes, **caractérisée en ce que** le pH de la composition est compris entre 4 et 11.

21. Procédé de teinture de fibres kératiniques humaines, **caractérisé en ce que** l'on applique sur lesdites fibres une composition tinctoriale selon l'une quelconque des revendications 1 à 20, et une composition oxydante comprenant au moins un agent oxydant : la composition oxydante étant mélangée au moment de l'emploi à la composition tinctoriale ou appliquée séquentiellement sans rinçage intermédiaire.

22. Dispositif à plusieurs compartiments pour la teinture de fibres kératiniques humaines, comportant au moins un premier compartiment contenant une composition tinctoriale selon rune quelconque des revendications 1 à 20 et un deuxième compartiment contenant une composition oxydante comprenant au moins un agent oxydant.

## Claims

1. Dye composition in liquid form, the viscosity of which is not more than 150 m.Pas (150 cPs) at a temperature of 25°C under 1 atmosphere, measured using a RheoStress 1 rheometer, under a shear rate of 200 s⁻¹, free of fatty acid or of fatty acid salts, comprising at least one oxidation dye and 2-methyl-1,3-propanediol.

2. Composition according to the preceding claim, **characterized in that** the content of 2-methyl-1,3-propanediol represents 0.5% to 20% by weight, more particularly 5% to 20% by weight and preferably 10% to 18% by weight of the dye composition.

3. Composition according to either of the preceding claims, **characterized in that** it comprises at least one polyol containing at least two hydroxyl radicals, other than 2-methyl-1,3-propanediol and/or a polyol monoether or polyether, the polyol and the monoether or polyether comprising a saturated or unsaturated, linear or branched hydrocarbon-based radical containing 3 to 50 carbon atoms and advantageously from 3 to 10 carbon atoms, optionally interrupted with one or more oxygen atoms.

4. Composition according to the preceding claim, **characterized in that** the polyol is chosen from pinacol, glycerol, 1,2,3-butanetriol, 3-methyl-1,3,5-pentanetriol, 1,2,4-butanetriol, propylene glycol, dipropylene glycol, 1,5-pentanediol, 3-methyl-1,5-pentanediol, neopentyl glycol, isoprene glycol and hexylene glycol, or mixtures thereof.

5. Composition according to the preceding claim, **characterized in that** the polyol is chosen from glycerol, propylene glycol, dipropylene glycol and hexylene glycol, or mixtures thereof.

6. Composition according to any one of Claims 3 to 5, **characterized in that** the proportion of polyol is such that the weight ratio of 2-methyl-1,3-propanediol/polyol is greater than 1 and preferably less than or equal to 10.

7. Composition according to any one of the preceding claims, **characterized in that** it comprises at least one short-chain monoalcohol, more particularly at least one monoalcohol comprising a linear on branched alkyl chain, containing from 2 to 8 carbon atoms.

8. Composition according to the preceding claim, **characterized in that** the content of short-chain monoalcohol is between 0.1% and 20% by weight relative to the weight of the dye composition, preferably between 0.2% and 20% by weight relative to the weight of the dye composition.

9. Composition according to any one of the preceding claims, **characterized in that** the oxidation dye is chosen from oxidation bases and/or couplers.

10. Composition according to the preceding claim, **characterized in that** the oxidation base is chosen from ortho-phenylenediamines, para-phenylenediamines, double bases, ortho-aminophenols, para-aminophenols and heterocyclic bases, and also the addition salts thereof with an acid or an alkaline agent, alone or as mixtures.

11. Composition according to the preceding claim, **characterized in that** the content of oxidation bases is between 0.0005% and 12% by weight relative to the weight of the dye composition, preferably between 0.005% and 8% by weight relative to the weight of the dye composition.

12. Composition according to Claim 9, **characterized in that** the couplers are chosen from meta-aminophenols, meta-phenylenediamines, meta-diphenols, naphthols and heterocyclic couplers, and the addition salts thereof with an acid or an alkaline agent.

13. Composition according to the preceding claim, **characterized in that** the content of couplers is between 0.0001% and 10% by weight relative to the weight of the dye composition, preferably between 0.005% and 5% by weight relative to the weight of the dye composition.

14. Composition according to any one of the preceding claims, **characterized in that** it comprises at least one direct dye.

15. Composition according to the preceding claim, **characterized in that** the direct dye is chosen from nitrobenzene dyes, azo dyes, anthraquinone, naphthoquinone or benzoquinone dyes, indigoid dyes or triarylmethane-based dyes, alone or as mixtures.

16. Composition according to either of Claims 14 and 15, **characterized in that** the content of direct dye represents from 0.0005% to 12% by weight relative to the weight of the dye composition, preferably from 0.005% to 6% by weight relative to the weight of the dye composition.

17. Composition according to any one of the preceding claims, **characterized in that** it comprises at least one nonionic, cationic, amphoteric or zwitterionic surfactant.

18. Composition according to the preceding claim, **characterized in that** the surfactant content is between 0.01% and 40% by weight relative to the weight of the dye composition, preferably between 0.1% and 30% by weight relative to the weight of the dye composition.

19. Composition according to any one of the preceding claims, **characterized in that** the composition comprises water.

20. Composition according to any one of the preceding claims, **characterized in that** the pH of the composition is between 4 and 11.

21. Process for dyeing human keratin fibres, **characterized in that** a dye composition according to any one of Claims 1 to 20, and an oxidizing composition comprising at least one oxidizing agent, are applied to the said fibres; the oxidizing composition being mixed at the time of use with the dye composition or applied sequentially without intermediate rinsing.

22. Multi-compartment device for dyeing human keratin fibres, comprising at least one first compartment containing a dye composition according to any one of Claims 1 to 20, and a second compartment containing an oxidizing composition comprising at least one oxidizing agent.

## Patentansprüche

1. Farbmittelzusammensetzung in Form einer Flüssigkeit, deren mit einem Rheometer Rheostress 1 unter einem Schergrad von 200 s⁻¹ gemessene Viskosität bei einer Temperatur von 25 °C und unter einer Atmosphäre höchstens 150 mPa (150 cP) beträgt und die keine Fettsäure oder Fettsäuresalze und mindestens einen Oxidationsfarbstoff und das 2-Methyl-1,3-propandiol enthält.

2. Zusammensetzung nach dem vorhergehenden Anspruch, **dadurch gekennzeichnet, dass** der Mengenanteil des 2-Methyl-1,3-propandiols 0,5 bis 20 Gew.-%, insbesondere 5 bis 20 Gew.-% und vorzugsweise 10 bis 18 Gew.-% der Farbmittelzusammensetzung ausmacht.

3. Zusammensetzung nach einem der vorhergehenden Ansprüche, **dadurch gekennzeichnet, dass** sie mindestens ein Polyol, das mindestens zwei Hydroxygruppen aufweist und das von 2-Methyl-1,3-propandiol verschieden ist, und/oder einen Monoether oder Polyether eines Polyols enthält, wobei das Polyol und der Mono- oder Polyether eine Kohlenwasserstoffgruppe umfassen, die gesättigt oder ungesättigt, geradkettig oder verzweigt ist und 3 bis 50 Kohlenstoffatome und vorteilhaft 3 bis 10 Kohlenstoffatome aufweist und die gegebenenfalls durch ein oder mehrere Sauerstoffatome unterbrochen ist.

4. Zusammensetzung nach dem vorhergehenden Anspruch, **dadurch gekennzeichnet, dass** das Polyol unter Pinacol, Glycerol, 1,2,3-Butantriol, 3-Methyl-1,3,5-Pentantriol, 1,2,4-Butantriol, Propylenglycol, Dipropylenglycol, 1,5-Pentandiol, 3-Methyl-1,5-pentandiol, Neopentylglycol, Isoprenglycol und Hexylenglycol oder deren Gemischen ausgewählt ist.

5. Zusammensetzung nach dem vorhergehenden Anspruch, **dadurch gekennzeichnet, dass** das Polyol unter Glycerol, Propylenglycol, Dipropylenglycol, Hexylenglycol oder deren Gemischen ausgewählt ist.

6. Zusammensetzung nach einem der Ansprüche 3 bis 5, **dadurch gekennzeichnet, dass** der Mengenanteil des Polyols so ist, dass das Gewichtsverhältnis 2-Methyl-1,3-propandiol/Polyol größer 1 und vorzugsweise kleiner oder gleich 10 ist.

7. Zusammensetzung nach einem der vorhergehenden Ansprüche, **dadurch gekennzeichnet, dass** sie mindestens einen kurzkettigen Monoalkohol und insbesondere mindestens einen Monoalkohol enthält, der eine geradkettige oder verzweigte Alkylgruppe mit 2 bis 8 Kohlenstoffatomen aufweist.

8. Zusammensetzung nach dem vorhergehenden Anspruch, **dadurch gekennzeichnet, dass** der Mengenanteil des kurzkettigen Monoalkohols im Bereich von 0,1 bis 20 Gew.-%, bezogen auf das Gesamtgewicht der Farbmittelzusammensetzung, und vorzugsweise im Bereich von 0,2 bis 20 Gew.-%, bezogen auf das Gesamtgewicht der Farbmittelzusammensetzung, liegt.

9. Zusammensetzung nach einem der vorhergehenden Ansprüche, **dadurch gekennzeichnet, dass** der Oxidationsfarbstoff unter den Oxidationsbasen und/oder Kupplern ausgewählt ist.

10. Zusammensetzung nach dem vorhergehenden Anspruch, **dadurch gekennzeichnet, dass** die Oxidationsbasen einzeln oder in Form von Gemischen unter den o-Phenylendiaminen, p-Phenylendiaminen, Doppelbasen, o- Aminophenolen, p-Aminophenolen, heterocyclischen Basen sowie den Additionssalzen dieser Verbindungen mit einer Säure oder einem alkalischen Stoff ausgewählt sind.

11. Zusammensetzung nach dem vorhergehenden Anspruch, **dadurch gekennzeichnet, dass** der Mengenanteil der Oxidationsbasen im Bereich von 0,0005 bis 12 Gew.-%, bezogen auf das Gesamtgewicht der Farbmittelzusammensetzung, und vorzugsweise im Bereich von 0,005 bis 8 Gew.-%, bezogen auf das Gesamtgewicht der Farbmittelzusammensetzung, liegt.

12. Zusammensetzung nach Anspruch 9, **dadurch gekennzeichnet, dass** die Kuppler unter den m-Aminophenolen, m-Phenylendiaminen, m-Dihydroxybenzolen, Naphtholen, heterocyclischen Kupplern und den Additionssalzen dieser Verbindungen mit einer Säure oder einem alkalischen Stoff ausgewählt sind.

13. Zusammensetzung nach dem vorhergehenden Anspruch, **dadurch gekennzeichnet, dass** der Mengenanteil der Kuppler im Bereich von 0,0001 bis 10 Gew.-%, bezogen auf das Gesamtgewicht der Farbmittelzusammensetzung, und vorzugsweise 0,005 bis 5 Gew.-%, bezogen auf das Gesamtgewicht der Farbmittelzusammensetzung, liegt.

14. Zusammensetzung nach einem der vorhergehenden Ansprüche, **dadurch gekennzeichnet, dass** sie mindestens einen Direktfarbstoff enthält.

15. Zusammensetzung nach dem vorhergehenden Anspruch, **dadurch gekennzeichnet, dass** der Direktfarbstoff einzeln oder in Form von Gemischen unter den nitro Benzolfarbstoffen, Azofarbstoffen, Anthrachinon-Farbstoffen, Naphthochinon-Farbstoffen oder Benzochinon-Farbstoffen, Indigoiden oder von Triarylmethan abgeleiteten Farbstoffen ausgewählt ist.

16. Zusammensetzung nach einem der Ansprüche 14 oder 15, **dadurch gekennzeichnet, dass** der Mengenanteil des Direktfarbstoffes 0,0005 bis 12 Gew.-%, bezogen auf das Gesamtgewicht der Farbmittelzusammensetzung, und vorzugsweise 0,005 bis 6 Gew.-%, bezogen auf das Gesamtgewicht der Farbmittelzusammensetzung, ausmacht.

17. Zusammensetzung nach einem der vorhergehenden Ansprüche, **dadurch gekennzeichnet, dass** sie mindestens einen nichtionischen, kationischen, amphoteren oder zwitterionischen grenzflächenaktive Stoff enthält.

18. Zusammensetzung nach dem vorhergehenden Anspruch, **dadurch gekennzeichnet, dass** der Mengenanteil des grenzflächenaktiven Stoffes im Bereich von 0,01 bis 40 Gew.-%, bezogen auf das Gesamtgewicht der Farbmittelzusammensetzung, und vorzugsweise im Bereich von 0,1 bis 30 Gew.-%, bezogen auf das Gesamtgewicht der Farbmittelzusammensetzung, liegt.

19. Zusammensetzung nach einem der vorhergehenden Ansprüche, **dadurch gekennzeichnet, dass** die Zusammensetzung Wasser enthält.

20. Zusammensetzung nach einem der vorhergehenden Ansprüche, **dadurch gekennzeichnet, dass** der pH-Wert der Zusammensetzung im Bereich von 4 bis 11 liegt.

21. Verfahren zum Färben von menschlichen Keratinfasern, **dadurch gekennzeichnet, dass** eine Farbmittelzusammensetzung nach einem der Ansprüche 1 bis 20 und eine oxidierende Zusammensetzung, die mindestens ein Oxidationsmittel enthält, auf die Keratinfasern aufgebracht werden, wobei die oxidierende Zusammensetzung bei der Anwendung mit der Farbmittelzusammensetzung vermischt wird oder anschließend ohne zwischenzeitliches Spülen aufgebracht wird.

22. Vorrichtung mit mehreren Abteilungen zum Färben von menschlichen Keratinfasern, die zumindest eine erste Abteilung, die eine Farbmittelzusammensetzung nach einem der Ansprüche 1 bis 20 enthält, und eine zweite Abteilung aufweist, die eine oxidierende Zusammensetzung mit mindestens einem Oxidationsmittel enthält.
